# EUROPEAN PATENT SPECIFICATION

(11) **EP 1 315 530 B1**
(45) Date of publication and mention of the grant of the patent: **02.11.2006**
(21) Application number: 00964613.4
(22) Date of filing: 07.09.2000
(51) Int. Cl.: A61M 1/36, A61M 39/02

(54) **AN INFUSION AND INFUSION/SAMPLING DEVICE FOR BLOOD CIRCUITS OUTSIDE THE BODY**
INFUSIONSVORRICHTUNG UND INFUSIONS- UND ENTNAHMEVORRICHTUNG FÜR EXTRAKORPORALE BLUTKREISLÄUFE
INFUSION ET DISPOSITIF D'INFUSION/D'ECHANTILLONNAGE POUR CIRCUITS SANGUINS SITUES HORS DU CORPS

(43) Date of publication of application: 04.06.2003
(73) Proprietor: Fresenius Medical Care Deutschland GmbH, 61352 Bad Homburg v.d.H. (DE)
(72) Inventor: PORRO, Giampiero, I-22100 Como (IT); REITER, Reinhold, I-26013 Crema (IT)
(74) Representative: Dragotti, Gianfranco
(86) International application number: PCT/IT2000/000355
(87) International publication number: WO 2002/020072

(56) References cited:
- WO-A-92/21403
- FR-A- 2 772 280
- US-A- 5 306 265
- US-A- 5 370 624

## Description

The present invention relates in general to devices for the infusion of injectable solutions by haematic or enteral routes and, more specifically, to disposable devices of the above-mentioned type associated with parenteral infusion sets or directly with devices for circulation outside the body.

The infusion of injectable solutions, particularly of a pharmacological nature, is a practice which is very widespread in the medical field for the treatment of various pathological conditions; for example, this practice is used for nutritional support when pathological conditions are present in the digestive system, in post-operative situations, for the treatment of states of shock and, more generally, in all cases in which it is necessary to introduce into the circulatory system drugs or substances which cannot be assimilated if administered by other routes.

The infusion is typically performed by introducing a needle into the selected blood vessel; the rear end of the needle is connected to a portion of flexible, tubular, plastics material which in turn is connected to the container of the solution to be infused into the patient. If the container is disposed at a level above the point of infusion into the patient, the solution to be injected flows by gravity and passes into the venous circulatory system.

Other devices and components having specific functions are normally connected in the tubular element, between the container of the solution and the infusion needle, that is:
- a dripping chamber which serves as a visual check of the infusion taking place and as a small reserve of the solution being infused;
- a flow-regulator based on more or less pronounced squashing of the tubular element and also permitting instantaneous discontinuance of the supply;
- an "infusion point" which enables another solution to be added to the primary infusion solution.

The infusion point is typically Y-shaped and comprises a main cylindrical portion from which a lateral portion or branch extends, the lateral portion also being cylindrical and its free or distal end being connectible to the container of the additional solution; the distal end of the main cylindrical portion is connected to the main infusion needle by means of the lower tubular section, and the proximal end portion of the main cylindrical element is closed by a perforable diaphragm. This diaphragm is usually made of synthetic rubber or natural rubber latex.

When the main infusion solution is to be supplemented by an additional solution, the above-mentioned rubber diaphragm is perforated by a conventional syringe filled with the additional solution. When the syringe is withdrawn after the additional solution has been injected into the flow of the main infusion solution, the opening created by the needle closes by virtue of the resilience of the rubber of the diaphragm so that the injection point can be re-used for the administration of further additional solutions or even for administering various medications to the patient without the need to subject him to separate injections.

In pathological conditions which require blood circulation outside the body, as in the case of haemodialysis and heart surgery, there is also a need to infuse medications into a patient.

As is known, during circulation outside the body, a blood-flow is created from the patient to the treatment apparatus (a haemodialyzer or a heart-lung machine) and back to the patient, by means of a suitable set of tubes.

In this case also, the blood circuit usually has one or more injection points and sampling points which enable samples of the blood in circulation to be withdrawn, for example, for analytical purposes, these latter points being substantially similar to the infusion points except that, in this case, the syringe serves for withdrawing blood samples.

In circulation outside the body, however, tubular elements of larger diameter are used, since the flow-rates of the liquids involved are greater and the dimensions of the infusion and sampling points are therefore also correspondingly modified or their shape is even changed. For example, whereas in intravenous infusion the injection point is usually Y-shaped, in the case of circuits outside the body, a T-shaped injection or sampling point (usually known as a "needle point") is preferred, but nevertheless still involves the presence of a diaphragm of resilient material which can be perforated upon injection or sampling.

The "needle point" consists of a cylindrical body, usually made of biocompatible plastics material, having an axial passage for the main blood-flow when the cylindrical body is connected in the main blood circuit. The cylindrical body has a sleeve-like radial projection in the region of which a radial opening is formed in the wall of the cylindrical body. This opening in turn is closed by a perforable septum of resilient material, preferably biocompatible synthetic rubber, the septum being held in place by a ring nut engaged by screwing (or by another method such as, for example, snap-engagement or ultrasound welding) with the outer surface of the sleeve-like projection.

In order to perform the infusion or sampling, the surface of the septum is perforated by the needle of a syringe the end of which passes through the radial hole in the cylindrical body and is positioned in the main blood flow. The infusion of the solution contained in the syringe into the main blood flow or the withdrawal of a metered quantity of blood into the above-mentioned cylinder is effected by operating the plunger of the syringe in a direction such as to expel the liquid contained in the cylinder of the syringe, or in a direction such as to draw liquid into the cylinder in question.

In recent years, however, the use of injection or sampling points of the above-mentioned type, that is, with the use of a syringe for the perforation of the diaphragm, has raised ever increasing problems since their use inevitably leads to the risk to medical and paramedical personnel of accidental pricking, thus exposing the personnel to serious risks of infections and contagions, mainly of a viral nature, which are transmissible by means of the blood.

Precisely in order to prevent these problems connected with the use of syringes, various devices which have the same functional purpose but which use syringes or the like without needles have already been proposed and developed.

Amongst these solutions there are three which are used mainly in blood lines for infusion combined with the taking of blood samples.

The first of these solutions provides for a modification of the "needle point" device by the replacement of the normal perforable septum with one which has a pre-cut slot so that it is possible to perform sampling or infusions without the aid of a needle but simply by a suitable tip of plastics material fitted on the syringe instead of the normal steel needle.

The second solution provides for the use of a three-way tap which comprises, substantially, a cylindrical seat having three radial openings arranged at predetermined angular spacings (usually at 90°).

A cylindrical or male element of the valve, housed in the cylindrical seat, is rotatable about its axis (and hence about the axis of the cylindrical seat) and has three radial ducts converging in the centre and opening in the surface of the cylindrical element in positions offset angularly in the same manner as the openings formed in the cylindrical seat.

Usually, with the openings offset by 90°, two of these ducts are aligned along a single axis coinciding with the path of the main blood flow and the third duct, which is arranged perpendicular to the axis of the first two, constitutes the route for the introduction of solutions to be infused.

If the cylindrical male element is rotated through an angle of less than 90°, it is possible to close the main blood circuit completely, whereas a rotation through 90° enables the above-mentioned third duct to be put into flow communication with one of the two branches of the main blood circuit.

The third solution provides for a combination known as a "T-piece" which is connected to the blood line in the region of or in place of the above-mentioned "infusion point" so as to incorporate the infusion and sampling functions at the same level.

This device consists of a conventional "needle point" mounted in the blood line and enabling blood samples to be taken; a tubular infusion duct engaged thereon has a manual clamp for stopping and/or regulating the flow, above which a male or female Luer-lock connector is fitted, with the function of putting the blood circuit into communication with the infusion set. Upstream of the Luer-lock connector there is a safety cap which prevents accidental and inadvertent opening of the manual clamp without first of all having connected the infusion line, leading to an accidental loss of blood flow.

For the second and third solutions, however, the results achieved appear unsatisfactory both from the safety point of view and from the point of view of the complexity of the operations to be performed in sequence, which lead to a high chance of errors on the part of the personnel involved.

For example, in the case of the three-way tap, the personnel involved often confuse the identifications of the lines put into communication, which confusion may in turn lead to loss of blood.

In the case of the "T-piece" the tubular portion corresponds to a dead space in which blood may stagnate, forming clots when the infusion line is not in use.

Moreover, the duplication of safety elements (the cap and the clamp in the case of the T-piece) is detrimental to speed of operation in emergencies.

Devices purely for the infusion or injection of additional solutions, of the type with "non-return valves", have also been proposed in the past and are substantially similar to the "needle points" described briefly above except that the opening for the penetration of the tip of the needle of the infusion syringe into the main axial duct (meaning that intended for the main blood flow) after the perforation of the closure septum is closed instead by a membrane provided with a hole which communicates with an upstream chamber only when the membrane is sufficiently deformed by the pressure of the injection or infusion of the liquid to be introduced into the main blood flow. Naturally, this solution can be used solely for infusion operations and not for taking blood samples.

WO / A / 92 / 21403 discloses an infusion device for introduction of nutrition solutions in an extra-corporeal blood circuit.

A cylindrical tubular body has an axial passage for blood and, a perpendicular cylindrical seat on the tubular body communicating with the passage through a radial opening.

In the seat is housed a valve body which can be opened and closed by rotation of a connecting unit engaged with the side walls of the seat.

The connecting unit has an axial duct for nutrition solutions aligned vertically with the centre of the radial opening.

It therefore remains desirable to provide infusion and/or infusion/sampling devices, usable in particular for blood circuits outside the body, which are substantially free of the problems and disadvantages mentioned briefly above.

More generally, it seems desirable to provide infusion and/or infusion/sampling devices for blood circuits which are not only free of the above-mentioned problems and disadvantages but which meet all of the requirements which these devices should ideally fulfil, which requirements are summarized briefly below.
(i) The materials with which the devices are made should be chemically and biologically non-toxic and should have good compatibility with blood; in this connection, as well as using biocompatible plastics materials for all of the components of the device, medical grade silicone rubbers and thermoplastic elastomers are particularly to be recommended for the hydraulic sealing components.
(ii) The devices should be suitable for being sterilized by the most common and widely accepted sterilization methods (ethylene oxide, steam and irradiation with beta/gamma radiation); the use of olefin polymers (such as polyethylene and polypropylene) for the external components, of polycarbonate or ABS for the components directly in contact with the blood-flow and with the solutions to be infused, and of silicone rubber for the seals is compatible with all of the above-mentioned sterilization methods.
(iii) Specific components such as syringes with Luer cones, Luer-locks and infusion lines with connectors of the same Luer type, in both male and female versions, should be easily connectible to the device.
(iv) The design of the devices should be such as to minimize turbulence and stagnation in the blood line, to exclude blood stagnation in the device, and to permit sampling of blood and infusion of other liquids with high flow-rates (up to about 600 cc/min.).
(v) The device should permit a safety closure which excludes any contamination and also prevents leakages of blood from the blood circuit, both during connection and disconnection to the device, and as a result of accidental operations.
(vi) The device should be versatile in the sense of permitting both the infusion of liquids with high flow-rates and the withdrawal of blood samples without the use of syringes or other components which are dangerous to the personnel involved, and also of ensuring that the blood sample taken is not contaminated by previous samples and that, during the sampling, there is no risk of contamination of the blood-flow from which the sample is taken.
(vii) Finally, the devices should be compatible with the economic factors of industrial production since the disposable nature of the devices leads to considerable consumption thereof.

The objects of the present invention are achieved substantially by an infusion device for blood circuits outside the body, of the type comprising a cylindrical, tubular body which has an axial passage and is intended to be inserted in a blood-flow circuit in a manner such that the axial passage forms part of the path of the blood-flow, a seat formed by a cylindrical sleeve fixed rigidly to the tubular body in a manner such that the base or lower surface of the seat is tangential to the tubular body, the seat communicating with the tubular body through a radial opening formed in the wall or shell of the tubular body, a septum of resilient material housed in the seat, and a connecting and closure ring nut engaged with the side walls of the sleeve-like seat so as to clamp the septum in position against the base surface of the seat, the ring nut having an axial duct, aligned vertically with the centre of the radial opening and having an outer end which can be connected to a unit for the supply of infusion solution and an inner end or mouth open towards and freely communicating with the space between the septum and the lower surface of the ring nut, characterized in that each of the septum and the tubular body has at least one infusion hole for putting the axial passage of the tubular body into communication with the space between the septum and the lower surface of the ring nut, the lower surface of the ring nut being parallel to the base surface of the sleeve-like seat and hence to the septum, the mouth of the axial duct of the ring nut projecting from the lower surface so as to bear against the septum in a leaktight manner in the region of the radial opening, the lower surface of the ring nut also having a first circumferential recess concentric with the projecting mouth of the axial duct and at least one second recess extending from the first recess as far as the at least one infusion hole so that, in the condition in which infusion solution is supplied under a pressure greater than a predetermined threshold, an infusion path is defined from the axial duct of the ring nut to the at least one infusion hole.

As will be appreciated better from the following detailed description given with reference to the drawings, with the infusion device according to the present invention, it becomes possible to open communication between the blood-flow path and the exterior only when the hydraulic connection with the external supply of solution to be infused has been made and the supply is taking place at a pressure greater than the internal pressure of the main blood-flow.

Moreover, in the event of inadvertent or accidental detachment of the supply of solution to be infused, the device of the invention operates as a non-return valve, preventing any leakage or loss of blood to the exterior.

Equally important is the fact that the device has a single open position for performing the infusion so that human errors are not possible.

Finally, a "needle point" of the type described above, which can be associated with a sampling syringe operating without a needle, can be associated with the above-mentioned cylindrical body, of suitable dimensions.

With reference to the drawings:
Figure 1 is an axially-sectioned view of the version of the device of the present invention which is usable solely for infusion, in the open condition for performing the infusion,
Figure 2 is a perpendicular axial section of the device of Figure 1,
Figures 3 and 4 are similar views of the same device in the closed condition,
Figure 5 is a plan view of the ring nut of the device of the preceding drawings, from below,
Figure 6 is a plan view of the septum made of resilient material used in the device of Figures 1-4,
Figure 7 is a plan view of the cylindrical sleeve-like seat of the device of Figures 1-4, from above,
Figures 8 and 9 are views similar to Figures 1 and 2, of the version of the device of the invention which is usable for infusion/sampling.

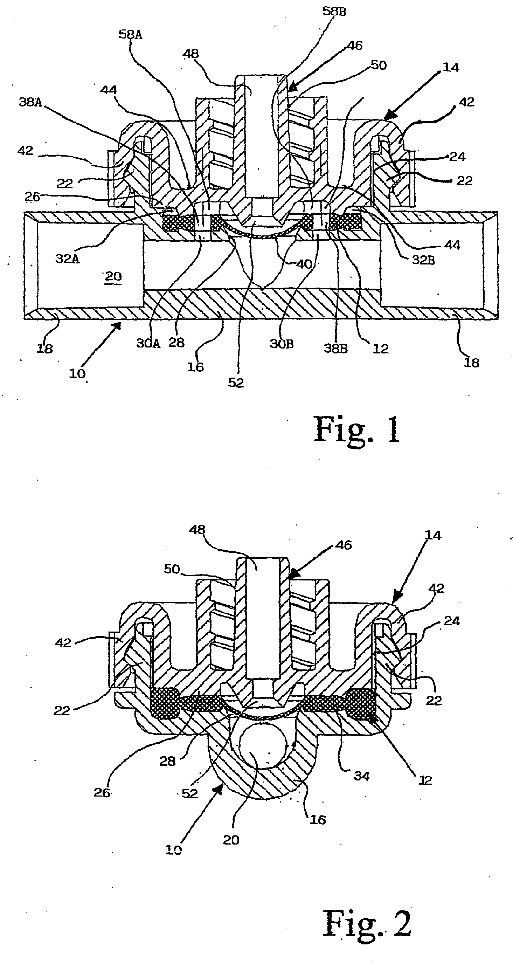

With reference first of all to Figures 1 and 2 as well as Figures 5, 6 and 7, these show an embodiment of the version of the device of the invention which is suitable for the infusion of a solution into the bloodstream circulating in a circuit outside the body.

The device comprises three main components, that is:
(a) a cylindrical, tubular body indicated as a whole and generally by the reference numeral 10,
(b) a septum 12 made of resilient material, and
(c) a connecting and closure ring nut, indicated as a whole by the reference numeral 14.

The cylindrical tubular body 10 shown in section in Figures 1 and 2 and in plan from above in Figure 7, comprises a duct 16 having two end connectors 18 for the engagement of the two ends of a circuit (not shown) for the circulation of blood outside the body. The axial passage for the circulation of the bloodstream is indicated 20.

Formed integrally with the tubular duct 16 which, as already stated, is made of biocompatible plastics material, is a sleeve 22 forming a substantially cylindrical seat 24 the base wall 26 of which is disposed in a plane tangential to the outer surface of the duct 16.

In the centre of the seat 24, in the wall of the duct 16, there is a radial opening 28 which puts the passage 20 into direct communication with the seat 24.

Moreover, in the region of the uppermost generatrix of the duct 16, there are two holes 30A and 30B which also put the passage 20 into communication with the space above the base wall 26.

The seat 24 also has, in the region of its peripheral boundary, two symmetrical steps 32A and 32B and two symmetrical recesses 33A and 33B having the functions explained below.

The septum 12 of resilient material such as, for example, biocompatible synthetic rubber, is housed in the seat 24 and has a circular body 34 having two curved recesses 36A and 36B for coupling with the steps 32A and 32B when the septum 12 is positioned against the base 26 of the seat 24 so as to prevent any translational or rotary movements of the septum relative to the seat 24 and, in particular, relative to its base 26.

Two through-holes, indicated 38A and 38B, are perfectly axially aligned with the holes 30A and 30B, respectively, when the septum 12 is fitted in the seat 24.

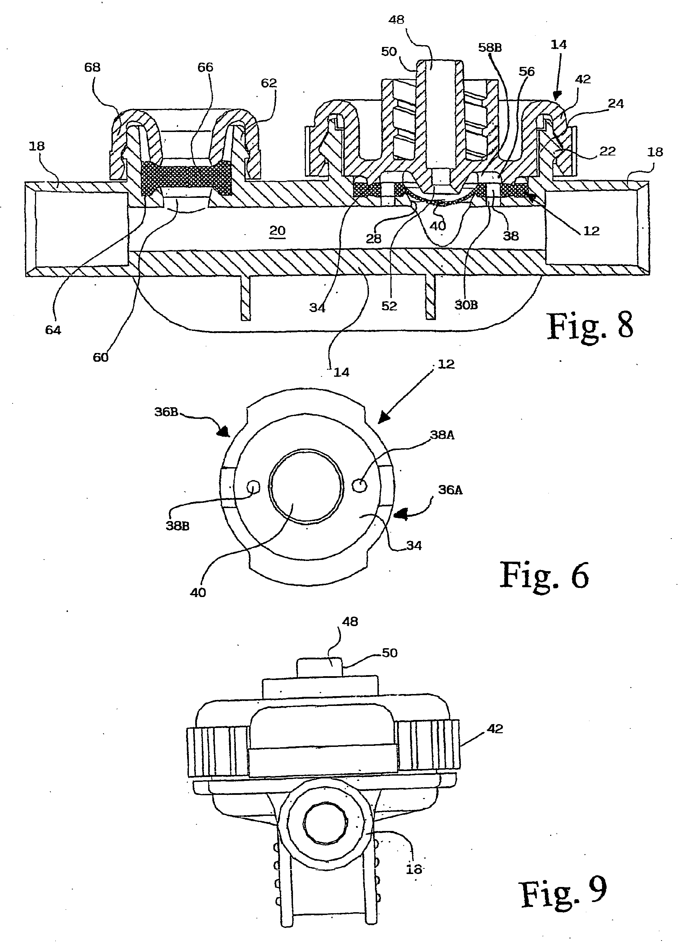

Finally, in Figure 6, the portion of the septum which is disposed in a centred position relative to the opening 28 is indicated 40.

With reference, finally, to the connecting and centring ring nut 14, this comprises a sleeve 42 which is U-shaped in section and embraces the vertical wall 22 of the sleeve forming the seat 24. The sleeve 42 is connected by means of a radial wall 44 to a connector, generally indicated 46. This connector has an axial duct 48, the outer wall 50 of which has threads to permit the coupling of a conventional infusion device, for example, of the Luer type, not shown in the drawings.

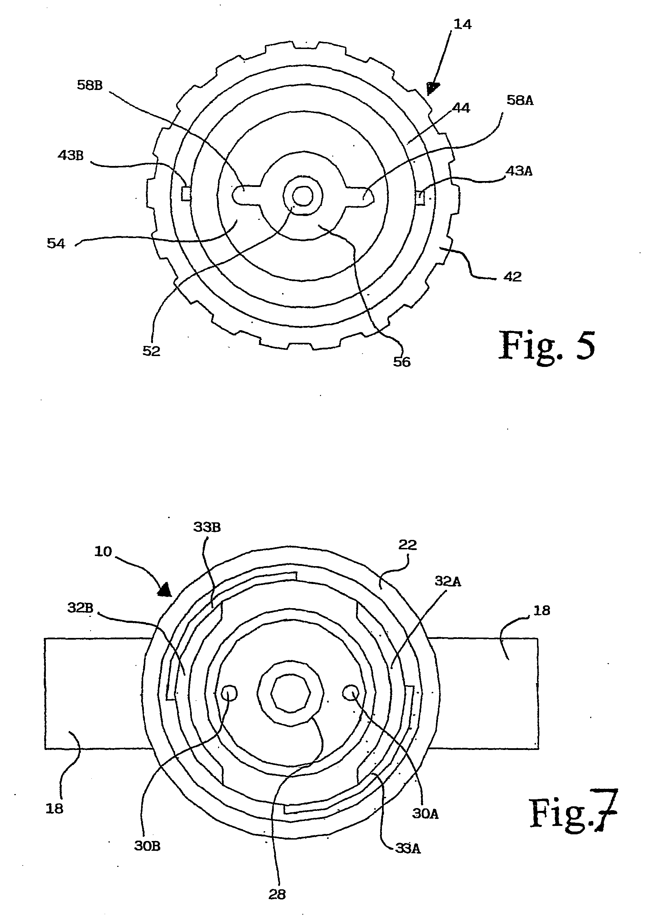

The lower wall of the ring nut 14, shown in plan in Figure 5, has a mouth 52 which constitutes the end of the axial duct 48, the mouth projecting a predetermined distance from the plane of the adjacent base wall 54 of the ring nut 14.

The base wall 54 has a first recess 56, concentric with the centre of the mouth 52, and two second recesses 58A and 58B disposed radially in the base wall 54 and extending from the first recess 56 as far as a point aligned axially with the holes 30A, 30B and 38A, 38B when the device is assembled.

By rotation of the ring nut 14 about the axis of the axial duct 48, it is possible to bring the two second recesses to positions in which they are aligned or are not aligned with the infusion holes 38A, 38B and hence with the holes 30A, 30B. Preferably, the recesses 33A and 33B are provided in the outer wall of the seat 26 and stop elements 43A and 43B engage therein so as to limit the rotation of the ring nut to the two above-mentioned positions, which positions are shown in Figures 1,2 and 3,4, respectively.

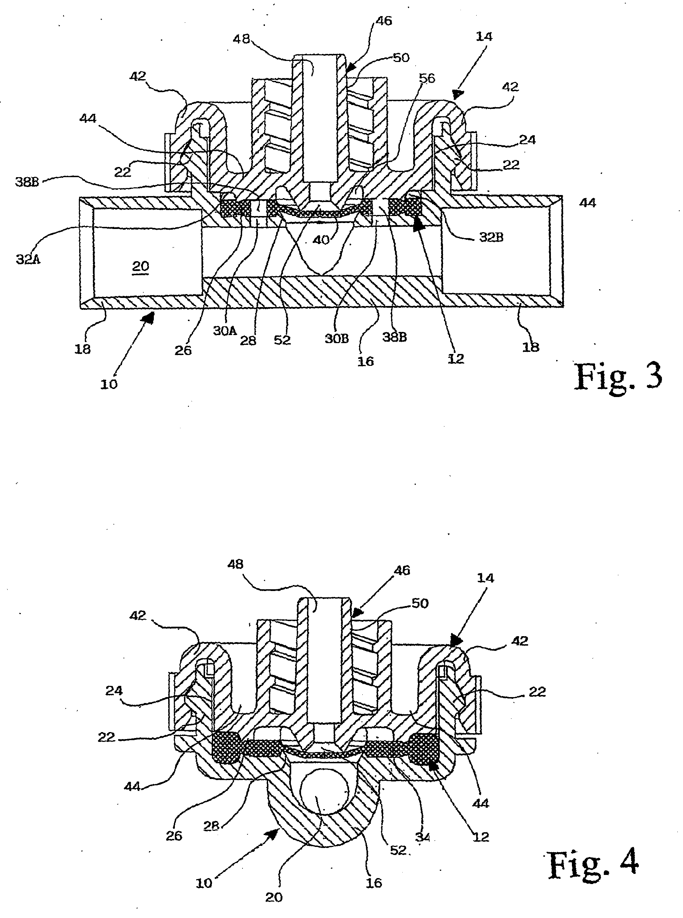

The device operates as follows: with the ring nut 14 arranged in the condition shown in Figures 3, 4, in which the connection is closed, the radial opening 28 is closed by the septum 12 and the second recesses 58A and 58B are not aligned with the holes 30A, 38A and 30B, 38B so that no communication between the axial duct 48 and the main blood-flow duct 20 is possible.

Once an infusion set has been connected to the axial duct 48 of the ring nut 14, the latter is rotated from the position of Figure 3 to that of Figure 1 so that the second recesses 58A, 58B are disposed in the region of the infusion holes 30A, 38A and 30B, 38B, naturally communicating at the other end with the first circumferential recess 56.

However, the communication between these second recesses and the axial duct 48 is still blocked owing to the fact that the mouth 52 of the axial duct 48 presses against the upper surface of the septum 12, thus forming a seal.

When the liquid to be infused is supplied into the duct 48 with sufficient pressure to overcome the pressure existing in the passage 20, the portion 40 of the septum 12 disposed in the region of the opening 38 is deformed, entering the opening and the passage 20, so that an open hydraulic connection is established between the mouth 52 of the axial duct 28, the first circumferential recess 56, the two second recesses 58A and 58B, and the corresponding pairs of infusion holes 30A, 38A and 30B, 38B, so that the solution to be infused is injected into the bloodstream flowing in the passage 20.

If, for any reason, the supply pressure of the solution to be infused in the axial duct 48 should be reduced below the threshold value or, in any case, below the pressure of the bloodstream, the septum 12 is immediately retracted against the mouth 52, interrupting the communication between the passage for the bloodstream and the axial duct 48 so that there is no appreciable loss of blood, except for the truly minimal quantity which may infiltrate into the injection holes, into the second recesses, and into the first recess.

It is thus clear that the primary object of the invention is achieved in a simple and safe manner.

With reference now to Figures 8 and 9, these show the embodiment of the invention which is usable both for infusion and for the withdrawal of samples of the bloodstream circulating in the passage 20.

In this embodiment, an infusion device identical to that shown in Figures 1 and 2 is shown in the right-hand portion of the drawing.

In this embodiment, the tubular element 16 provides a second hole 60 formed in its wall between the two connectors 18, this hole being defined by a sleeve-like element 62, also preferably formed integrally with the wall of the tubular element 16, in the outer surface of which a circular seat 64 is formed for housing a septum 66 in a position adjacent the hole 60.

The septum 66 is preferably made of resilient material similar to that forming the septum 12 of the infusion device and is held in position by a ring nut 68 having a U-shaped wall so as to embrace from the exterior the cylindrical wall of the sleeve 62 to which it is coupled, for example, by a screw thread.

The septum 66 is preferably of the pre-perforated type which allows the end of a nozzle or tip fixed to a sampling syringe to be inserted.

It can easily be appreciated from Figure 8 that the combined device illustrated therein enables the main body constituted by the tubular duct 16, the two end connectors 18, and the two sleeves 22 and 62 to be manufactured by moulding.. At the same time, still at the moulding stage, it is possible to pre-form the holes 28 and 60 as well as, in the case of the infusion device, the holes 30A and 30B, the steps 32A and 32B and, finally, the seats 33A and 33B.

The septa 12 and 66 are then produced separately, provided with the holes 38A, 38B and the recessed portions 36A, 36B in the case of the septum 12, and with the pre-perforation known *per se* in the case of the septum 66.

Finally, the two ring nuts 42 and 68 are produced, again separately.

The device can be assembled easily and quickly and its operation, as has been seen, is safe, is not susceptible to human error, and is free of dangers to personnel involved with regard to the taking of samples of the blood-flow.

Moreover, any blood leakages or stagnations of blood are substantially eliminated.

Moreover, all of the requirements listed above for devices of this type can be and are complied with and fulfilled, both from the point of view of the materials usable and from the point of view of the infusion of other liquids with high flow rates and the taking of blood samples without contamination of the samples taken, or of the main blood-flow, by previous samples.

The invention has been described with reference to preferred embodiments but, naturally, conceptually and structurally equivalent modifications and variations are possible and to be expected without departing from the scope of the claims.

## Claims

1. An infusion device for blood circuits outside the body, comprising a cylindrical, tubular body (16) which has an axial passage (20) and is intended to be inserted in a blood-flow circuit in a manner such that the axial passage forms part of the path of the blood-flow, a seat (24) formed by a cylindrical sleeve (22) fixed rigidly to the tubular body in a manner such that the base or lower surface (26) of the seat is tangential to the tubular body, the seat (24) communicating with the tubular body (16) through a radial opening (28) formed in the wall or shell of the tubular body, a septum (12) of resilient material, housed in the seat (24), and a connecting and closure ring nut (14) engaged with the side walls of the sleeve-like seat so as to clamp the septum (12) in position against the base surface (26) of the seat (24), the ring nut (14) having an axial duct (48) aligned vertically with the centre of the radial opening (28) and having an outer end which can be connected to a unit for the supply of infusion solution and an inner end or mouth (52) open towards and freely communicating with a space between the septum (12) and a lower surface (54) of the ring nut, and wherein each of the septum (12) and the tubular body (16) has at least one infusion hole (38 and 30, respectively) for putting the axial passage (20) of the tubular body (16) into communication with the space between the septum (12) and the lower surface (54) of the ring nut, the lower surface of the ring nut being parallel to the base surface (26) of the sleeve-like seat and hence to the septum, the mouth (52) of the axial duct (48) of the ring nut (14) projecting from the lower surface (54) of the ring nut so as to bear against the septum (12) in a leaktight manner in the region of the radial opening (28), the lower surface (54) of the ring nut also having a first circumferential recess (56) concentric with the projecting mouth (52) of the axial duct (48) and at least one second recess (58) extending from the first recess (56) as far as the at least one infusion hole (30, 38), so that, in a condition in which infusion solution is supplied under a pressure greater than a predetermined threshold into the axial duct (48), an infusion path is defined from the axial duct (48) of the ring nut to the at least one infusion hole (38, 30).

2. An infusion device for blood circuits outside the body according to Claim 1, **characterized in that** respective pairs of diametrally aligned holes (30A, 30B and 38A, 38B) are formed in the septum (12) and in the base surface (26) of the seat (24) and two second radial recesses (58A, 58B) are correspondingly formed in the base wall (54) of the ring nut (14).

3. An infusion device for blood circuits outside the body according to Claim 1, **characterized in that** the predetermined pressure threshold is greater than the pressure present in the blood-flow circulating in the axial passage (20) of the tubular duct (16).

4. An infusion device for blood circuits outside the body according to Claim 1, **characterized in that** two symmetrical steps are formed in the base surface (26) of the seat (24) and the septum (12) has peripheral recessed portions or indentations (36A, 36B) for coupling with the steps.

5. An infusion device for blood circuits outside the body according to Claim 1, **characterized in that** the base surface (26) of the seat (24) has two symmetrical recesses (33A, 33B) and the base surface (54) of the ring nut (14) has two stop elements (43A, 43B) for cooperating with the recesses (33A, 33B) in order to limit the angle of rotation of the ring nut relative to the sleeve-like seat between the open and closed positions of the device.

6. An infusion device for blood circuits outside the body according to Claim 1, **characterized in that** the axial duct (48) of the ring nut (14) has an outer surface (50) having means for temporary coupling with a device for the supply of infusion solution.

7. An infusion/sampling device for blood circuits outside the body, **characterized in that** a second cylindrical sleeve-like portion (62) is fixed to the shell of the cylindrical tubular body (16) of the infusion device according to Claim 1, the base of the second cylindrical sleeve-like portion (62) being in communication with the axial passage (20) of the tubular duct (16) through a radial hole (60), the radial hole being defined by a circular seat (64) formed in the sleeve, the seat (64) being able to house a septum (66) of resilient material, held in position by a ring nut (68) in locking engagement with the sleeve-like portion (62), the septum (66) having means for the withdrawal of blood from the bloodstream circulating in the axial passage (20).

8. An infusion/sampling device for blood circuits outside the body according to Claim 7, **characterized in that** the septum (66) is pre-perforated for sampling by means of a nozzle or a tip fixed to a syringe without a needle.

9. An infusion/sampling device for blood circuits outside the body according to Claim 7, **characterized in that** the locking nut has a U-shaped side wall so as to embrace the cylindrical wall of the sleeve (62) from the exterior.

## Patentansprüche

1. Infusionsvorrichtung für Blutkreisläufe außerhalb des Körpers mit
einem zylindrischen, rohrförmigen Körper (16), der einen axialen Durchgang (20) aufweist und dazu bestimmt ist, so in einen Blutflusskreis eingesetzt zu werden, das der axiale Durchgang einen Teil des Pfads für den Blutfluss bildet,
einem Sitz (24), der von einer zylindrischen Hülse (22) gebildet wird, die starr an dem rohrförmigen Körper befestigt ist, so dass die Basis oder Grundfläche (26) des Sitzes tangential zu dem rohrförmigen Körper ist, wobei der Sitz (24) mit dem rohrförmigen Körper (16) über eine radiale Öffnung (28) in Verbindung steht, die in der Wand oder dem Mantel des rohrförmigen Körpers gebildet ist,
einer Scheidewand (12) aus einem nachgiebigen Material, die in dem Sitz (24) untergebracht ist, und
einer Verbindungs- und Verschlussringmutter (14), die mit den Seitenwänden des hülsenartigen Sitzes in Eingriff ist, so dass die Scheidewand (12) in einer Stellung gegen die Grundfläche (26) des Sitzes (24) geklemmt wird;
wobei die Ringmutter (14) einen axialen Durchgang (48) aufweist, der vertikal mit der Mitte der radialen Öffnung (28) ausgerichtet ist und ein äußeres Ende aufweist, das mit einer Einheit zum Zuführen einer Infusionslösung verbunden werden kann, sowie ein inneres Ende oder einen Mund (52), der offen ist zu und frei in Verbindung steht mit einem Zwischenraum zwischen der Scheidewand (12) und einer Grundfläche (54) der Ringmutter, und
wobei die Scheidewand (12) und der rohrförmige Körper (16) beide zumindest ein Infusionsloch (38 bzw. 30) aufweisen, um den axialen Durchgang (20) des rohrförmigen Körpers (16) mit dem Zwischenraum zwischen der Scheidewand (12) und der Grundfläche (54) der Ringmutter in Verbindung zu bringen,
die Grundfläche (54) der Ringmutter parallel zu der Grundfläche (26) des hülsenartigen Sitzes und somit zu der Scheidewand ist,
der Mund (52) des axialen Durchgangs (48) der Ringmutter (14) von der Grundfläche (54) der Ringmutter aus vorspringt, so dass er in dem Bereich der radialen Öffnung (28) leckdicht gegen die Scheidewand (12) drückt,
die Grundfläche (54) der Ringmutter auch eine erste umlaufende Vertiefung (56) aufweist, die konzentrisch zu dem vorspringenden Mund (52) des axialen Durchgangs (48) ist, und zumindest eine zweite Vertiefung (58), die sich von der ersten Vertiefung (56) aus bis zu dem zumindest einen Infusionsloch (30, 38) hin erstreckt, so dass in einem Zustand, in dem in den axialen Durchgang (48) eine Infusionslösung unter einem Druck zugeführt wird, der größer als eine vorbestimmte Schwelle ist, ein Infusionspfad von dem axialen Durchgang (48) der Ringmutter aus bis zu dem zumindest einen Infusionsloch (38, 30) hin gebildet wird.

2. Infusionsvorrichtung für Blutkreisläufe außerhalb des Körpers nach Anspruch 1, **dadurch gekennzeichnet, dass**
in der Scheidewand (12) und in der Grundfläche (26) des Sitzes (24) jeweils Paare von diametral ausgerichteten Löchern (30A, 30B und 38A, 38B) ausgebildet sind und
dementsprechend in der Grundwand (54) der Ringmutter zwei zweite radiale Vertiefungen (58A, 58B) ausgebildet sind.

3. Infusionsvorrichtung für Blutkreisläufe außerhalb des Körpers nach Anspruch 1, **dadurch gekennzeichnet, dass** die vorbestimmte Druckschwelle größer ist als der Druck, der in dem Blutfluss herrscht, der durch den axialen Durchgang (20) des rohrförmigen Kanals (16) hindurchläuft.

4. Infusionsvorrichtung für Blutkreisläufe außerhalb des Körpers nach Anspruch 1, **dadurch gekennzeichnet, dass**
in der Grundfläche (26) des Sitzes (24) zwei symmetrische Stufen gebildet sind und
die Scheidewand Randvertiefungsabschnitte oder Einbuchtungen (36A, 36B) aufweist zum Kuppeln mit den Stufen.

5. Infusionsvorrichtung für Blutkreisläufe außerhalb des Körpers nach Anspruch 1, **dadurch gekennzeichnet, dass**
die Grundfläche (26) des Sitzes (24) zwei symmetrische Vertiefungen (33A, 33B) aufweist und
die Grundfläche (54) der Ringmutter (14) zwei Anschlagelemente (43A, 43B) aufweist zum Zusammenwirken mit den Vertiefungen (33A, 33B), um den Drehwinkel der Ringmutter relativ zu dem hülsenartigen Sitz zwischen dem geöffneten und dem geschlossenen Zustand der Vorrichtung zu begrenzen.

6. Infusionsvorrichtung für Blutkreisläufe außerhalb des Körpers nach Anspruch 1, **dadurch gekennzeichnet, dass** der axiale Durchgang (48) der Ringmutter (14) eine Außenfläche (50) aufweist, die ein Mittel zum temporären Verbinden mit einer Vorrichtung zum Zuführen einer Infusionslösung aufweist.

7. Infusions- und Entnahmevorrichtung für Blutkreisläufe außerhalb des Körpers, **dadurch gekennzeichnet, dass**
ein zweiter zylindrischer hülsenartiger Abschnitt (62) an dem Mantel des rohrförmigen Körpers (16) der Infusionsvorrichtung nach Anspruch 1 befestigt ist,
die Basis des zweiten zylindrischen hülsenartigen Abschnitts (62) über ein radiales Loch (60) mit dem axialen Durchgang (20) des rohrförmigen Körpers (16) in Verbindung steht,
das radiale Loch (60) von einem in der Hülse ausgebildeten kreisförmigen Sitz (64) begrenzt wird,
der Sitz (64) in der Lage ist, eine Scheidewand (66) aus einem nachgiebigen Material aufzunehmen, die von einer Ringmutter (68), die in verriegelndem Eingriff mit dem hülsenartigen Abschnitt (62) steht, in ihrer Stellung gehalten wird, und
die Scheidewand (66) ein Mittel aufweist zur Entnahme von Blut aus dem Blutstrom, der durch den axialen Durchgang (20) hindurchläuft.

8. Infusions- und Entnahmevorrichtung für Blutkreisläufe außerhalb des Körpers nach Anspruch 7, **dadurch gekennzeichnet, dass** die Scheidewand (66) vorperforiert ist für die Entnahme mittels einer Düse oder einer Spitze, die an einer Spritze ohne Nadel befestigt ist.

9. Infusions- und Entnahmevorrichtung für Blutkreisläufe außerhalb des Körpers nach Anspruch 7, **dadurch gekennzeichnet, dass** die Verriegelungsmutter eine U-förmige Seitenwand aufweist, so dass sie die zylindrische Wand der Hülse (62) von außen umgreift.

## Revendications

1. Dispositif d'infusion pour circuits sanguins extérieurs au corps, comprenant un corps tubulaire, cylindrique (16) ayant un passage axial (20) et destiné à être inséré dans un circuit de flux sanguin de manière que le passage axial forme une partie du chemin du flux sanguin, un siège (24) formé par un manchon cylindrique (22) fixé de manière rigide au corps tubulaire, de sorte que la base ou surface inférieure (26) du siège soit tangente au corps tubulaire, le siège (24) communiquant avec le corps tubulaire (16) à travers une ouverture radiale (28) formée dans la paroi ou coque du corps tubulaire, une cloison (12) en matériau élastique, ménagée dans le siège (24) et un écrou à anneau de fermeture et de connexion (14) mis en prise avec les parois latérales du siège semblable à un manchon, de façon à fïxer la cloison (12) en position contre la surface de base (26) du siège (24), l'écrou à anneau (14) ayant un conduit axial (48) aligné verticalement avec le centre de l'ouverture radiale (28) et ayant une extrémité extérieure qui peut être connectée à une unité pour l'alimentation d'une solution d'infusion et une extrémité interne ou bouche (52) s'ouvrant vers et communiquant librement avec un espace entre la cloison (12) et une surface inférieure (54) de l'écrou à anneau, et dans lequel chacun des éléments parmi la cloison (12) et le corps tubulaire (16) a au moins un orifice d'infusion (38 et 30 respectivement) pour mettre le passage axial (20) du corps tubulaire (16) en communication avec l'espace entre la cloison (12) et la surface inférieure (54) de l'écrou à anneau, la surface inférieure de l'écrou à anneau étant parallèle à la surface de base (26) du siège semblable à un manchon et donc à la cloison, la bouche (52) du tuyau axial (48) de l'écrou à anneau (14) se projetant depuis la surface inférieure (54) de l'écrou à anneau, de façon à porter contre la cloison (12) de manière étanche dans la zone de l'ouverture radiale (28), la surface inférieure (54) de l'écrou à anneau ayant également un premier évidement circonférentiel (56) concentrique avec la bouche de projection (52) du tuyau axial (48) et au moins un second évidement (58) s'étendant depuis le premier évidement (56) jusqu'à au moins un trou d'infusion (30, 38), de sorte que, dans une condition pour laquelle la solution d'infusion est alimentée sous une pression supérieure à un seuil prédéterminé dans le tuyau axial (48), une voie d'infusion est définie depuis le tuyau axial (48) de l'écrou à anneau vers au moins un orifice d'infusion (38, 30).

2. Dispositif d'infusion pour circuits sanguins extérieurs au corps selon la revendication 1, **caractérisé en ce que** les paires respectives d'orifices diamétralement alignés (30A, 30B et 38A, 38B) sont ménagées dans la cloison (12) et dans la surface de base (26) du siège (24) et deux seconds évidements radiaux (58A, 58B) sont formées de manière correspondante dans la paroi de base (54) de l'écrou à anneau (14).

3. Dispositif d'infusion pour circuits sanguins extérieurs au corps selon la revendication 1, **caractérisé en ce que** le seuil de pression prédéterminé est supérieur à la pression présente dans le flux de sang circulant dans le passage axial (20) du conduit tubulaire (16).

4. Dispositif d'infusion pour circuits sanguins extérieurs au corps selon la revendication 1, **caractérisé en ce que** deux épaulements symétriques sont formés dans la surface de base (26) du siège (24) et la cloison (12) a des portions creuses périphériques ou indentations (36A, 36B) à coupler avec les épaulements.

5. Dispositif d'infusion pour circuits sanguins extérieurs au corps selon la revendication 1, **caractérisé en ce que** la surface de base (26) du siège (24) a deux évidements symétriques (33A, 33B) et la surface de base (54) de l'écrou à anneau (14) a deux éléments d'arrêt (43A, 43B) pour coopérer avec les évidements (33A, 33B), afin de limiter l'angle de rotation de l'écrou à anneau relativement au siège semblable à un manchon entre les positions ouverte et fermée du dispositif.

6. Dispositif d'infusion pour circuits sanguins extérieurs au corps selon la revendication 1, **caractérisé en ce que** le tuyau axial (48) de l'écrou à anneau (14) a une surface extérieure (50) ayant des moyens à coupler temporairement avec un dispositif pour l'alimentation d'une solution d'infusion.

7. Dispositif d'infusion/échantillonnage pour circuits sanguins extérieurs au corps, **caractérisé en ce qu'**une seconde portion semblable à un manchon cylindrique (62) est fixée à la coque du corps tubulaire cylindrique (16) du dispositif d'infusion selon la revendication 1, la base de la seconde portion semblable à un manchon cylindrique (62) étant en communication avec le passage axial (20) du conduit tubulaire (16) à travers un orifice radial (60), l'orifice radial étant défini par un siège circulaire (64) formé dans le manchon, le siège (64) étant en mesure d'abriter une cloison (66) en matériau élastique, maintenue en position par un écrou à anneau (68) en prise de verrouillage avec la portion semblable à un manchon (62), la cloison (66) ayant des moyens pour prélever du sang du flux sanguin circulant dans le passage axial (20).

8. Dispositif d'infusion/échantillonnage pour circuits sanguins extérieurs au corps selon la revendication 7, **caractérisé en ce que** la cloison (66) est pré-perforée pour prélever au moyen d'une buse ou d'une pointe fixée à une seringue sans aiguille.

9. Dispositif d'infusion/échantillonnage pour circuits sanguins extérieurs au corps selon la revendication 7, **caractérisé en ce que** l'écrou de verrouillage a une paroi latérale en forme de U, de façon à encercler la paroi cylindrique du manchon (62) depuis l'extérieur.
